# EUROPÄISCHE PATENTSCHRIFT

(11) **EP 0 597 359 B1**
(45) Veröffentlichungstag und Bekanntmachung des Hinweises auf die Patenterteilung: **29.09.1999**
(21) Anmeldenummer: 93117747.1
(22) Anmeldetag: 02.11.1993
(51) Int. Cl.: G01N 33/545, C12Q 1/00, C12N 11/08

(54) **Verfahren zur Durchführung immundiagnostischer Nachweise**
Process for the performance of immunodiagnostic assays
Procédé pour la mise en oeuvre d'essais immunodiagnostiques

(30) Priorität: 13.11.1992 DE 4238389
(43) Veröffentlichungstag der Anmeldung: 18.05.1994
(73) Patentinhaber: BAYER AG, 51368 Leverkusen (DE)
(72) Erfinder: Brandt, Heinz-Dieter, Dr., D-47800 Krefeld (DE); Dhein, Rolf, Dr., D-47800 Krefeld (DE); Hildenbrand, Karlheinz, Dr., D-47802 Krefeld (DE); Stöcker, Roland, Dr., D-40724 Hilden (DE)

(56) Entgegenhaltungen:
- EP-A- 0 186 347
- EP-A- 0 225 535
- WO-A-86/07345
- CHEMICAL ABSTRACTS, Band 107, Nr. 21, 23. November 1987, Columbus, Ohio, USA P. HERMANN et al. "Immobili- zation of proteins on macro- porous polymeric carriers: use of salts to increase covalent coupling." Seite 389, Nr. 194 164c; & DD-A-242 240

## Beschreibung

Die vorliegende Erfindung betrifft ein Verfahren zur Durchführung immundiagnostischer Nachweise, bei welchen Enzyme oder Antikörper auf poröse Trägermaterialien gebunden werden. Das erfindungsgemäße Verfahren ist gekennzeichnet durch den Einsatz makroporöser Polymermembranen als solche porösen Trägermaterialien. Die makroporösen Polymermembranen werden hergestellt, indem man aus einer Lösung, die wenigstens zwei unverträgliche Polymere in solchen Mengen, die zu einer Phasentrennung in der Lösung führen, enthält, durch Eindispergieren eines unlöslichen Füllstoffes, eine homogene Gießlösung bereitet, diese Lösung auf einen Träger aufbringt und dann eine Fällungskoagulation durchführt. Hierbei weisen die Polymermembranen eine durch freie Aminogruppen modifizierte Oberfläche auf, die durch den Zusatz von Polyethylenimin oder von iminmodifizierten Füllstoffen zur Gießlösung erhalten wird.

Poröse Trägermaterialien, die biologisch aktive Proteine, wie Enzyme oder Antikörper, binden, spielen in verschiedenen Bereichen der Technik sowie in der klinischen Diagnostik eine wichtige Rolle. Den Immunoassay-Methoden kommt dabei eine besondere Bedeutung zu. Am meisten werden kompetitive Bindungstests (RIA, EIA) sowie Sandwich-Tests (IRMA, ELISA) benutzt, wobei die ELISA-Tests eine zunehmende Bedeutung erlangen. Beim ELISA-Test (Enzyme Linked Immunosorbent Assay) wird das zu bestimmende Antigen von einem trägergebundenen Antikörper (primärer Antikörper) gebunden und durch einen enzymatischen Zweitantikörper (sekundärer Antikörper) quantitativ bestimmt.

Das Grundprinzip dieser Nachweismethoden ist in zahlreichen Publikationen beschrieben (Angew. Chemie 97 (1985), 141-163; Chimia 29, (3) (1975), 109). Die Immobilisierung (Bindung) des primären Antikörpers an der porösen Matrix-Oberfläche kann prinzipiell auf zwei unterschiedliche Arten erfolgen, nämlich durch Adsorption oder kovalente Bindung, wobei beide Verfahren Vor- und Nachteile besitzen. Während bei der Adsorption die Gefahr der Denaturierung der biochemischen Wirkstoffe gering ist, besteht bei der Testdurchführung die Gefahr des Auswaschens oder Ausblutens; beim kovalenten Verfahren ist es gerade umgekehrt. Deshalb wird es angestrebt, je nach Anwendungsbeispiel und Testverfahren alternativ (sowohl adsorptiv als auch kovalent) verfahren zu können.

Der Nachweis der immunchemischen Testreaktion erfolgt beim ELISA-Verfahren häufig durch eine chromogene Reaktion, die durch ein Enzym, das sich am sekundären Antikörper befindet, katalysiert wird. So werden beispielsweise häufig sekundäre, mit Peroxydase (POD) markierte Antikorper eingesetzt, die in Gegenwart von H₂O₂ eine Oxidationsreaktion auslösen, wobei ein Farbumschlag erfolgt. Das Chromogen wird im Laufe der Testprozedur in die Matrix imprägniert, wobei im Falle von POD-katalysierten Reaktionen häufig das bekannte wasserlösliche Trinder-System 4-Aminoantipyrin/2-Hydroxy-3,5-dichlorbenzol-sulfonat (H.V. Bergmeyer, Methods of Enzymatic Analysis, Vol. I, 3rd. ed., Verlag Chemie) verwendet wird.

Andere Enzym-/Chromogen-Systeme sind: Alkalische Phosphatase/p-Nitrophenylphosphat oder β-Galactosidase/o-Nitrophenylgalactosid. Auf dem Markt werden Membransysteme für die Immundiagnose beispielsweise von den Firmen Millipore (Produkt: Immobilon®) oder PALL (Produkt: Immunodyne®) in Kombination mit Broschüren, die den Testablauf beschreiben, angeboten. Der Gesamttest besteht aus mehreren Inkubations- und Waschschritten, wobei die Fixierung des primären Antikörpers in der Regel durch kovalente Bindung erfolgt. Zur Testdurchführung werden Mikrotiterplatten oder Dot-Blot-Apparaturen, beispielsweise von der Fa. Bio Rad angeboten. Kritisch bei der Testdurchführung sind Auswasch- und Ausblutprobleme, wobei im Rahmen von mehreren Imprägnier- und Waschschritten die zuvor eingebrachten Substanzen und Wirkstoffe wieder ausgewaschen werden können. Das ist insbesondere kritisch bei den bisher eingesetzten Chromogenen, die in der Regel nicht kovalent gebunden werden können und wegen ihrer oft guten Wasserlöslichkeit bei den anschließenden Waschprozeduren beträchtlich extrahiert werden.

Dadurch werden die Meßergebnisse häufig verfälscht und die Empfindlichkeit reduziert.

Es war daher wünschenswert, Membranmatrizen für immunchemische Nachweisreaktionen zur Verfügung zu haben, die sich besonders für das ELISA-Verfahren eignen und die folgende Merkmale aufweisen:
a) Möglichkeit sowohl zu adsorptiven als auch kovalenten Immobilisierung,
b) einfache und schonende Möglichkeit zur kovalenten Immobilisierung und
c) verbesserte Fixierung des Chromogens zur weitgehenden Verhinderung des Ausblutens der Reaktionsfarben.

Es wurde nun überraschenderweise gefunden, daß makroporöse Membranen aus Polymer-Blends, die in DE-OS 38 09 523 beschrieben sind, hervorragend für Immundiagnostische Nachweisverfahren geeignet sind.

Es wurde ein Verfahren zur Durchführung immundiagnostischer Nachweise, bei welchen Enzyme oder Antikörper auf porösen Trägermaterialien gebunden werden, gefunden, das dadurch gekennzeichnet ist, daß als poröse Trägermaterialien makroporöse Polymermembranen eingesetzt werden, die hergestellt werden, indem man
a) in eine Lösung, die wenigstens zwei unverträgliche Polymere in solchen Mengen, die zu einer Phasentrennung in der Lösung führen, enthält, unlösliche Füllstoffe eindispergiert, wobei eine homogene Gießlösung entsteht, und
b) diese Lösung auf einen Träger aufbringt und eine Fällungskoagulation durchführt, wobei die Polymermembranen eine durch freie Aminogruppen modifizierte Oberfläche aufweisen, die durch den Zusatz von Polyethylenimin oder von aminomodifizierten Füllstoffen zur Gießlösung erhalten wird.

Werden beispielsweise eine 20 Gew.-%ige Lösung von Polyurethan in Dimethylformamid (PU/DMF-Lösung) und eine 20 Gew.-%ige Lösung von Polyacrylnitril in Dimethylformamid (PAN/DMF-Lösung) unter Rühren vermischt, so kommt es nach kurzem Stehen zur Phasenseparation. Derartige Gemische sind instabil und als Gießlösungen für die Membranherstellung nicht geeignet.

Vereinigt man dagegen dieselben Polymer/DMF-Lösungen unter gleichzeitigem oder nachträglichem Eindispergieren von Füllstoffen, beispielsweise von Talkum, so erhält man homogene, stabile Gießlösungen, die für die Membran; herstellung nach der Methode der Fällungskoagulation geeignet sind. Die aus derartigen Gießlösungen hergestellten Membranen zeigen überraschenderweise im Vergleich zu den bekannten deutlich größere Poren an der Oberfläche, eine sehr viel höhere Gesamtporosität und eine deutlich erhöhte Saugfähigkeit, die vergleichbar ist mit der der Chromatographiepapiere.

Elektronenmikroskopische Aufnahmen vom Querschnitt dieser Polymermembranen zeigen, daß es sich um Strukturen mit einem filzartigen Aufbau handelt, während der asymmetrische Strukturaufbau mit der dichten Polymerhaut an der Membranoberfläche fast vollständig zurückgedrängt ist. An der Membranoberfläche sind bei einer Membran des beschriebenen Aufbaus Porendurchmesser von bis zu 30 µm zu erkennen.

Die zur Herstellung derartiger makroporöser Membranmatrizen erforderlichen Polymergießlösungen müssen die folgenden Bedingungen erfüllen:
- Die Lösungen der einzelnen Polymerkomponenten dürfen nicht miteinander mischbar sein. Bei miteinander mischbaren Systemen werden in Analogie zu bisher bekannten Gießlösungen mikroporöse Membranstrukturen mit ausgeprägter asymmetrischer Struktur erhalten;
- die Lösungsmittel der einzelnen Polymerkomponenten müssen miteinander mischbar sein;
- um die nicht mischbaren Polymerkomponenten in homogene Gießlösungen zu überführen, müssen geeignete unlösliche, beispielsweise anorganische Füllstoffe eindispergiert werden.

Als Füllstoffe kommen beispielsweise in Frage: Talkum, Titandioxid, Bariumsulfat, Siliciumdioxid, mikrokristalline Zellulose, Zeolithe, Bentonite, Calciumcarbonat, Magnesiumcarbonat, Zinkoxid, Eisenoxide, bevorzugt Talkum, Titandioxid, Bariumsulfat, hochdisperse Kieselsäure (beispielsweise Aerosile, Fa. Degussa), Quarz, mikrokristalline Zellulose, Zeolithe und Bentonite. Derartige anorganische und organische Füllstoffe sind marktgängig. Nicht alle genannten Füllstoffe sind für alle Polymerkombinationen gleichermaßen geeignet. So sind beispielsweise Gießlösungen aus Polyurethan/Polyacrylnitril-Gemischen mit Titandioxid oder Bariumsulfat weniger stabil und zeigen eine erkennbare Inhomogenität, während Lösungen aus derselben Polymerkombination mit Talkum eine gute Homogenität und Dispersionsstabilität ergeben. Es können auch Gemische mehrerer der genannten Füllstoffe eingesetzt werden. Die Oberfläche der eingesetzten Füllstoffe kann ggf. organisch modifiziert sein. Beispielsweise sind hydrophobierte hochdisperse Kieselsäuren (Aerosil R 972®, Fa. Degussa) oder aminmodifizierte Typen kommerziell erhältlich.

Die Menge der eingesetzten Füllstoffe ist abhängig von deren spezifischen Oberflächen, liegt jedoch allgemein im Bereich von 5 bis 500 Gew.-%, bezogen auf das Gesamtgewicht der organischen Substanz der Membran. Bei hochdispersen Füllstoffen, beispielsweise Aerosil 200® (200 m²/g) sind Mengen im Bereich von 5 bis 50 Gew.-%, bezogen auf das Gewicht der organischen Substanz der Membran, ausreichend. Bei Füllstoffen mit geringeren spezifischen Oberflächen im Bereich von einigen m²/g kann die eingesetzte Füllstoffmenge im Bereich von 35 % bis 500 %, bezogen auf das Gewicht der organischen Substanz der Membran, liegen. Der Vorzugsbereich für hochdisperse Füllstoffe liegt bei 10 bis 25 Gew.-%, der für Füllstoffe mit geringeren spezifischen Oberflächen bei 50 bis 200 Gew.-%.

Als Polymerkombinationen kommen beispielsweise die folgenden in Frage:
- Celluloseester/Polyvinylester
- Polyurethan/Polyacrylderivate bzw. Acrylcopolymere
- Polycarbonat-Copolymere/Polyurethan
- Polyvinylderivate/Polysulfone
- Polyamide bzw. Polyimide/Polystyrol bzw. Styrolcopolymere
- Poly-para-dimethyl-phenylenoxid/Polyvinylidenfluorid
- Polysulfon/Polyacrylnitril
- Polyethersulfon / anionisch modifiziertes Polyacrylnitril.

Es sind auch weitere Kombinationen aus den genannten Polymersystemen möglich, die weiterhin zu ternären Polymergemischen erweitert werden können.

Konkrete Beispiele solcher Polymerkombinationen sind:
Celluloseacetat/Polyvinylacetat (z.B. Mowilith®), Polyurethan (Desmoderm KBH®)/Polyacrylnitril (Dralon T®), Desmoderm KBH®/Aminmodifiziertes Dralon (Dralon A®), Desmoderm KBH®/Anionisch modifiziertes Dralon (Dralon U®), Polysulfon (Udel P 1700®)/Polyvinylidenfluorid, Polyethercarbonat/Desmoderm KBH®, Dralon U®/Mowilith®, Celluloseacetat/Dralon U®, Celluloseacetat/Dralon U®/ Polystyrol, Mowilith®/Desmoderm KBH®/ Polyvinylchlorid und Polyethersulfon (Ultrason E®) / Polyacrylnitril (Dralon X®).

Das für eine Phasenseparation ohne den eindispergierten Füllstoff erforderliche Mengenverhältnis der Copolymere in den jeweiligen Kombinationen ist durch einfache Vorversuche zu ermitteln.

Ein weiteres sehr wichtiges ternäres Polymersystem ist Desmoderm KBH®/Mowilith®/Dralon T®, wobei Dralon T® auch durch Dralon A® oder Dralon U® ersetzt sein kann. Die chemischen Strukturen der bevorzugt eingesetzten Polymeren sind im Anhang an die Ausführungsbeispiele beschrieben.

Zur Herstellung der Polymergießlösungen sind beispielsweise Dimethylformamid (DMF), N-Methylpyrrolidon (NMP), Dimethylsulfoxid (DMSO), Dimethylacetamid, Dioxolan, Dioxan, Aceton, Methylethylketon, Cellosolve® bzw. Gemische dieser Lösungsmittel geeignet.

Der Gesamtprozeß der Membranherstellung läßt sich anhand des folgenden Beispiels darstellen: Die jeweils ca. 20 Gew.-%igen DMF-Polymerlösungen aus Desmoderm KBH®, Mowilith® und Dralon® werden unter Eindispergieren von Talkum mit Hilfe eines schnelldrehenden Rührers (Dissolver) zu einer homogenen Polymergießlösung gemischt. Nach Entgasen im Vakuum wird die Gießlösung mit Hilfe eines Rakels in einer Schichtdicke von beispielsweise 150 µm auf ein Trägersubstrat aufgebracht und in das Koagulationsbad, vorzugsweise reines Wasser, getaucht. Nach einer Verweilzeit von etwa 2 Minuten wird die dabei entstandene Polymermembran aus dem Koagulationsbad genommen und mit warmer Luft getrocknet.

Weitere Komponenten für die Herstellung der makroporösen Membranen können Tenside, beispielsweise Dioctyl-natriumsulfosuccinat oder Dodecylbenzolsulfonate sein, die in der Gießlösung benutzt werden. Ebenso können wasserlösliche Polymere, wie Celluloseether, Polyethylenglykole, Polyvinylalkohol oder Polyvinylpyrrolidon Bestandteile der Polymergießlösung sein. Ferner kommen Zusätze von sogenannten Koagulationshilfsmitteln, wie beispielsweise kationische Polyurethandispersionen, in Frage.

Die Gießlösung wird auf einen Träger aufgebracht, um die Fällungskoagulation durchführen zu können und die Gießlösung und das makroporöse Polymer handhaben zu können. Solche Träger können beispielsweise Glas oder silikonisierte Trägermaterialien der verschiedensten Herkunft sein. Weitere Trägermaterialien sind flüssigkeitsdurchlässige Polymergewebe oder Polymervliese, auf denen die makroporöse Polymermembran eine gute Haftung zeigt.

Makroporöse Polymermembranen, die erfindungsgemäß eingesetzt werden, haben mittlere Porendurchmesser von 10 bis 50 µm, bevorzugt 10 bis 30 µm.

Besonders vorteilhaft bei der Durchführung immundiagnostischer Nachweise sind Membranen, in die das Chromogen bereits im Rahmen der Membranherstellung integriert wurde. Ein wichtiges Beispiel hierfür ist das Chromogen 3,3',5,5'-Tetramethyl-benzidin (TMB), das ein bekannter Oxidationsindikator ist. Gegenüber den bekannten konventionellen Systemen ergeben sich zwei wichtige Vorteile:
1. Die gesamte Testprozedur wird um den Schritt der Indikator-Imprägnierung verkürzt und damit vereinfacht. Dieser Aspekt ist insofern wichtig, da als Lösemittel für viele Indikatoren, beispielsweise auch für TMB, organische Lösungsmittel eingesetzt werden, die entweder die Polymermembran oder das biochemische Reagenzsystem schädigen können.
2. Ein noch gewichtigerer Vorteil des in die Membran integrierten Indikators ist der, daß das Ausbluten der bei der Reaktion erzeugten Indikatorfarbe im Gegensatz zu konventionellen Systemen praktisch vollkommen unterbunden wird.

Das erfindungsgemäße Verfahren unter Einsatz der beschriebenen makroporösen Polymermembranen ergibt überraschenderweise Möglichkeiten zur verbesserten adsorptiven und kovalenten Bindung von biochemischen Wirkstoffen (Enzymen, Antikörpern):

### a) Adsorptive Immobilisierung

Es zeigt sich überraschenderweise, daß die adsorptive Bindungseigenschaft der erfindungsgemäßen, durch an der Oberfläche mit freien Aminosäuren modifizierten makroporösen Polymermembranen erheblich gesteigert sind. Die Herstellung der aminmodifizierten Membranen erfolgt durch Zusätze in die Polymergießlösung, die zur Membranherstellung nach dem Verfahren der Fällungskoagulation in Wasser eingesetzt wird. Als geeignete Zusätze haben sich Polymere mit freien Aminogruppen, wie beispielsweise Polyethylenimin, erwiesen. Als weitere Zusätze kommen beispielsweise Polyamidamine oder andere wasserlösliche aminhaltige Polymere in Frage, wie sie beispielsweise auch als Retentionsmittel bei der Papierherstellung (Das Papier 33, Heft 10A) eingesetzt werden. Solche Retentionsmittel sind beispielsweise unter dem Namen Retaminol® (Bayer AG) oder Praestol® (Stockhausen) handelsüblich.

Obwohl solche Zusätze wasserlösliche Verbindungen darstellen, werden sie überraschenderweise im Verlauf der Membranherstellung durch Koagulation in Wasser nicht extrahiert. Diese Tatsache kann durch die Ninhydrin-Reaktion nachgewiesen werden.

Desweiteren wurde gefunden, daß die erfindungsgemäßen aminomodifizierten Membranen in einfacher Weise auch dadurch hergestellt werden können, daß man zur Polymergießlösung aminmodifizierte Füllstoffe zusetzt. Als solche kommen beispielsweise in Frage: Silikat-Füllstoffe, deren Oberfläche mit Aminosilan behandelt ist. Solche Produkte werden beispielsweise als Silbond 600 AST (Fa. Quartz-Werke) angeboten. Es hat sich dabei gezeigt, daß besonders gute Resultate im Hinblick auf die adsorptive Immobilisierung durch eine Kombination der Zusätze Polyethylenimin und mit Aminosilanen behandelten Silikaten erzielt werden.

### b) Kovalente Immobilisierung

Es wurde weiterhin gefunden, daß die beschriebenen erfindungsgemäßen aminmodifizierten makroporösen Polymermembranen auch für eine kovalente Fixierung der biochemischen Reagenzien hervorragend geeignet sind. Hierbei wurde überraschenderweise gefunden, daß neben den bekannten Kupplungsreagenzien, wie Glutardialdehyd, Carbodiimiden oder Bishydroxysuccinimiden, sich ganz besonders wasserlösliche Bisulfit-Addukte von mehrfunktionellen Isocyanaten als kovalente Ankergruppen eignen. Solche Bisulfit-Addukte und ihre Herstellung sind bekannt (Houben-Weyl, Methoden der organischen Chemie, Bd. 14/2, S. 63-64). Sie sind durch Umsetzung von Isocyanaten mit Natriumhydrogensulfit zugänglich. Geeignete Isocyanate sind vorzugsweise mehrfunktionell und können aliphatisch, cycloaliphatisch oder aromatisch sein. Beispiele geeigneLer Polyisocyanate sind: Hexamethylendiisocyanat, Cyclohexan-1,4-diisocyanat, 2,4-und 2,6-Toluylendiisocyanat und deren Gemische, 1-Isocyanatomethyl-5-isocyanato-1,3,3-trimethylcyclohexan, 2,2,4- bzw. 2,4,4-Trimethylhexamethylendiisocyanat-1,6, 1,5-Naphthalin-diisocyanat, 1,3-Cyclopentylendiisocyanat, m- und p-Phenylendiisocyanat, 2,4,6-Toluylentriisocyanat, 4,4',4"-Triphenylmethantriisocyanat, 1,3- und 1,4-Xylylendiisocyanat, 3,3'-Dimethyl-4,4'-diphenylen-diisocyanat, Duren-diisocyanat, 1-Phenoxy-2,4'-phenylendiisocyanat, 1-tert.-Butyl-2,4-phenylendiisocyanat, Methylen-bis-4,4'-cyclohexyldiisocyanat, 1-Chlor-2,4-phenylendiisocyanat und 4,4'- Diphenylenetherdiisocyanat; weitere, hier nicht genannte Polyisocyanate sind dem Fachmann grundsätzlich bekannt. Weiterhin ist es möglich, höhermolekulare und gegebenenfalls auch höherfunktionelle Polyisocyanate, die aus niedermolekularen Grundkörpern durch Polymerisationsreaktion zu Uretdionen oder Isocyanuratderivaten hergestellt werden, einzusetzen. Beispielsweise seien das Uretdion aus 2 Mol 2,4-Toluylendiisocyanat und diisocyanuratringhaltigen Polymerisationsprodukte aus 2,4- und 2,6-Toluylendiisocyanat oder Hexamethylendiisocyanat, ein durchschnittlich zwei Isocyanuratringe im Molekül enthaltendes und aus 5 Mol Toluylendiisocyanat gebildetes System oder ein entsprechendes Derivat aus durchschnittlich 2 Mol Toluylendiisocyanat und 3 Mol Hexamethylendiisocyanat, erwähnt. Nach einer weiteren Aufbaumethode ist es möglich, aus Di- oder Polyisocyanaten durch partielle Hydrolyse über die Stufe der Carbamidsäure und des Amins höhere biuretverknüpfte Systeme herzustellen, z.B. eine biuretverknüpfte Verbindung, die formal aus 3 Mol Hexamethylendiisocyanat unter Zusatz von 1 Mol Wasser und Abspaltung von 1 Mol Kohlendioxid entstanden ist. Ebenfalls geeignete Polyisocyanate erhalt man bei der Umsetzung von Di- und Polyolen mit di- oder polyfunktionellen Isocyanaten, wenn das Molverhältnis von Hydroxyverbindungen zum Isocyanat so gewählt wird, daß bei den statistisch gebildeten Reaktionsprodukten stets freie NCO-Funktionen vorhanden bleiben und ein Molgewicht von 2000 bis 3000 nicht überschritten wird. Ähnliche geeignete Polyisocyanate erhalt man aus hydroxylgruppenhaltigen Polyestern durch Umsetzen mit überschüssigen di- oder polyfunktionellen Isocyanaten. Alle oben beschriebenen Di- und Polyisocyanate können in dieser Weise mit Di-und Polyolen, wie Mono- und Polyethylenglykol, Propandiolen, Butandiolen, Neopentylglykol und anderen Pentandiolen, Adipol, Hexandiolen, Cyclohexandiolen, 1,4-Dihydroxymethylcyclohexan, Perhydro-bisphenol A, Glycerin, Trimethylolethan, Trimethylolpropan, anderen Hexantriolen und Pentaerythrit, unter den beschriebenen Voraussetzungen umgesetzt werden.

Bevorzugt werden Diisocyanate, insbesondere Hexamethylendiisocyanat, Isophorondiisocyanat, Toluylendiisocyanat und Diphenylmethan-diisocyanat eingesetzt.

Diese Methode der kovalenten Bindung von immunologisch aktiven Substanzen (Enzyme, Antikörper) auf der Membranoberfläche ist nicht nur überaus einfach, sondern sie bietet bezüglich der chemischen Struktur und der Kettenlänge der Bindungsglieder zwischen der porösen Polymermembran und den aktiven Substanzen eine bei den bisher bekannten Kupplungsreagenzien nicht bekannte Variationsbreite. Zur Durchführung der Bindung der aktiven Substanzen stehen mehrere Verfahren zur Verfügung.

So kann in einem ersten Verfahren die beschriebene Aminmodifizierte Membran zunächst mit einer etwa 0,1 bis 1%igen wäßrigen Lösung des Isocyanat-Bisulfit-Addukts getränkt werden. Dieses Tränken erfolgt beispielsweise sehr einfach durch Eintauchen der Membran in eine solche Lösung. Danach wird die Membran unter geeigneten Bedingungen getrocknet, beispielsweise in einem Trockenschrank bei 30 bis 80°C während einer Zeit von 5 bis 120 Minuten. Die Fixierung immunologisch aktiver Substanzen kann nun einfach durch Kontaktieren einer Lösung dieser Aktivsubstanzen mit der Membranoberfläche erfolgen. Das Kontaktieren geschieht beispielsweise einfach durch Imprägnierung oder beim Arbeiten mit Mikrotiterplatten oder Bio Dot-Apparaturen durch ein Durchsaugen der Wirkstofflösung.

In einem zweiten Verfahren wird eine gemeinsame wäßrige Lösung des Isocyanat-Bisulfit-Addukts und der zu immobilisierenden biochemisch aktiven Substanz hergestellt und diese mit der aminmodifizierten Membran in Kontakt gebracht. Hierbei hat sich ein Überschuß des BisulfitAddukts über die molar erforderliche Menge, bezogen auf die Aktivsubstanz, als vorteilhaft erwiesen.

Das erfindungsgemäße Verfahren erlaubt die gesamte Bandbreite immunchemischer Nachweismethoden, beispielsweise den immunologischen Nachweis von Pathogenen für Pflanze, Mensch und Tier, den Nachweis von Wirkstoffen in Pflanze, Mensch und Tier, den Nachweis von Verunreinigungen (beispielsweise von Pflanzenschutzmitteln) im Boden und Grundwasser und weitere Nachweismethoden.

Die Eignung des erfindungsgemäßen Verfahrens zur Durchführung solcher immunchemischer (immundiagnostischer) Nachweise ist in den folgenden Beispielen näher erläutert.

Die Beispiele beschreiben folgende Schritte:
1. Herstellung aminmodifizierter makroporöser Polymermembranen
2. Immundiagnostische Nachweisverfahren an den Beispielen Maus IgG
3. Vergleich zwischen adsorptiver und kovalenter Fixierung.

### Beispiel 1

### Herstellen einer aminmodifizierten Polymerblend-Membran

Mit Hilfe eines schnelldrehenden Rührers (Dissolvers) wurde aus den folgenden Komponenten eine Polymer-Gieß-lösung hergestellt:

| | |
|---|---|
| Dralon T® (Polyacrylnitril) | 9,6 g |
| Desmoderm KBH® (Polyurethan, 20 % in NMP) | 170,0 g |
| Mowilith 50® (Polyvinylacetat, 25 % in NMP) | 225,8 g |
| N-Methylpyrrolidon (NMP) | 220,0 g |
| Silbond 600 AST (aminmodifiziertes Silikat) | 293,4 g |
| Natriumdodecylsulfat (SDS) | 3,9 g |
| 3,3',5,5'-Tetramethylbenzidin | 31,3 g |
| Polymin P® (Polyethylenimin, 50 % in Wasser) | 2,1 g |

Die chemischen Strukturen der verwendeten Polymeren sind in DE-OS 3 809 523 beschrieben.

Die Gießlösung wurde filtriert (Metallgewebe, 25 µm Maschenweite) und entgast.

### Membranherstellung

Die Herstellung einer trägergestützten Membran erfolgte nach dem bekannten Verfahren der Fällungskoagulation in Wasser. Es wurden die folgenden Bedingungen eingehalten:

| | |
|---|---|
| Trägermaterial | Polyestervlies FO 2403, Fa. Freudenberg |
| Naßauftrag mit Rakel | 300 µm |
| Koagulationsbad | Wasser, 40°C |
| Trocknungsbedingungen | 60°C, 20 min |

Dabei wurde eine auf dem Polyestervlies haftende, den Indikator 3,3',5,5'-Tetramethylbenzidin enthaltende makroporöse Polymerblend-Membran erhalten, die als Trägermatrix für die folgenden immunchemischen Nachweisreaktionen eingesetzt wurde.

### Beispiel 2

Durchführung eines Sandwich-ELISA zum Nachweis von Maus IgG

Unter Verwendung kommerziell erhältlicher Immunglobuline wurde ein Immuntest nach dem Sandwich-ELISA-Prinzip auf der Polymerblend-Membran als Festphase durchgeführt. Die für den Immuntest verwendeten Komponenten, prim. (Fänger-) Antikörper, sek. (Marker-) Antikörper, Antigen (Maus IgG) wurden von der Firma SIGMA Chemie GmbH, Deisenhofen, bezogen.

Im einzelnen wurden folgende Antikörper verwendet:
1. als prim. Antikörper (Fängerantikörper):

| Sigma Nr. | Typ | Ursprung |
|---|---|---|
| M 3014 | a-Maus IgG ganzes Molekül | Ziege |
| M 9637 | a-Maus IgG ganzes Molekül | Kaninchen |

2. als sek. Antikörper (Markerantikörper):

| Sigma Nr. | Typ | Ursprung | Markierung |
|---|---|---|---|
| A 2028 | a-Maus IgG ganzes Molekül | Kaninchen | Peroxidase |
| A 4416 | a-Maus IgG ganzes Molekül | Ziege | Peroxidase |

3. als Antigen (Maus IgG)

| Sigma Nr. | Typ |
|---|---|
| I 5381 | Maus IgG |

Für Anwendungen wurden mehrere Kombinationen von prim. und sek. Antikörpern verwendet.

| Prim. Antikörper | Antigen | Sek. Antikörper |
|---|---|---|
| M 3014 | I 5381 | A 2028 |
| M 3014 | I 5381 | A 4416 |
| M 9637 | I 5381 | A 4416 |

Durchführung der Immuntestverfahren:

Als Rahmen für die Testmembran wurde eine Dot-Blot Apparatur von BioRad verwendet. Für die Filtrationsschritte wurde ein mildes Wasserstrahlvakuum angelegt.

Die Polymerblend-Membran wurde mit 100 µl H₂O je Dot gewässert. Danach wurde der prim. Antikörper mit einer Konzentration von 1 mg/ml Wasser in einem Volumen von 400 µl durch die Membran filtriert. Anschließend wurde eine Verdünnungsreihe des Antigens (Maus IgG) in einem Volumen von 200 µl je Verdünnung und unterschiedlicher Konzentration durch die Membran gesaugt. Nach einem Waschschritt wurde der sek. Antikörper (POD-markiert) durch die Membran filtriert. Abschließend wurde einmal mit 500 µl PBS/Tween und einmal mit 500 µl Wasser gewaschen und die Membran in die Substrat-Lösung für das Markerenzym zur Farbentwicklung getaucht.

Die Ergebnisse des Bindungsassay wurden in einem Taschen-Reflektometer bestimmt.

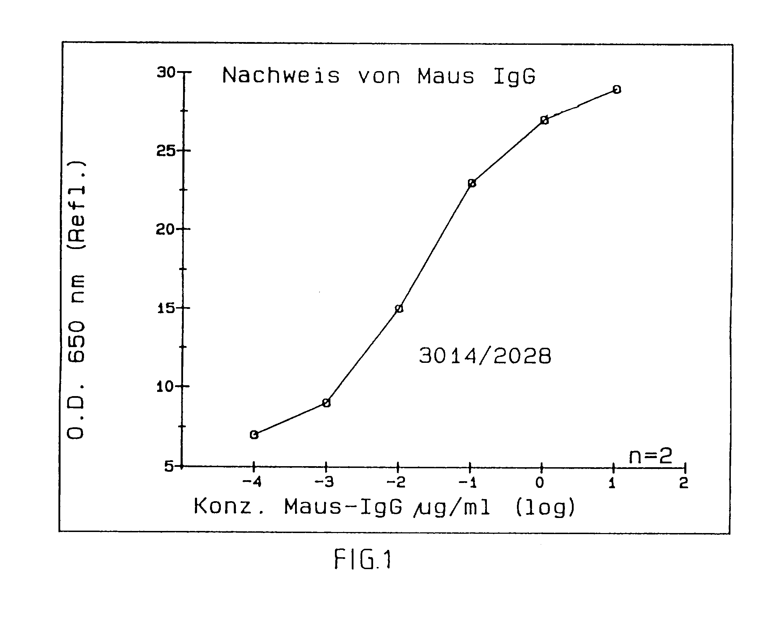

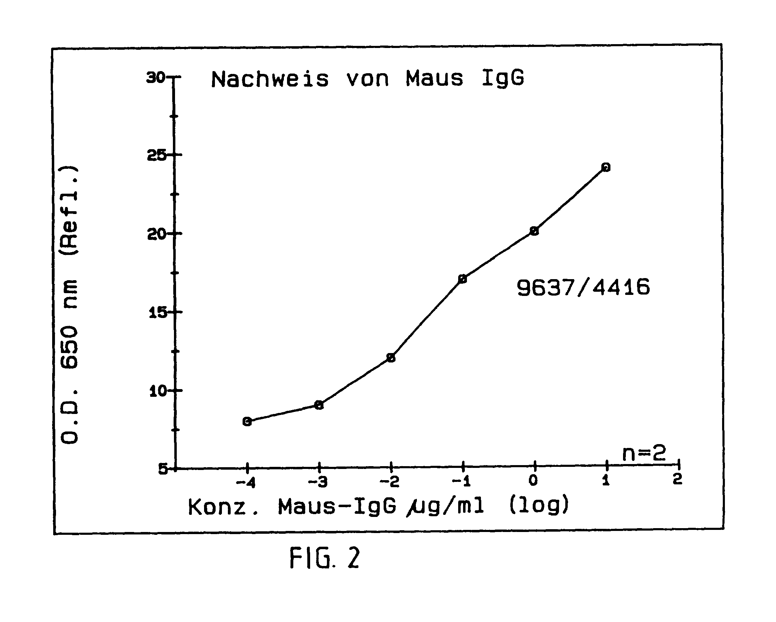

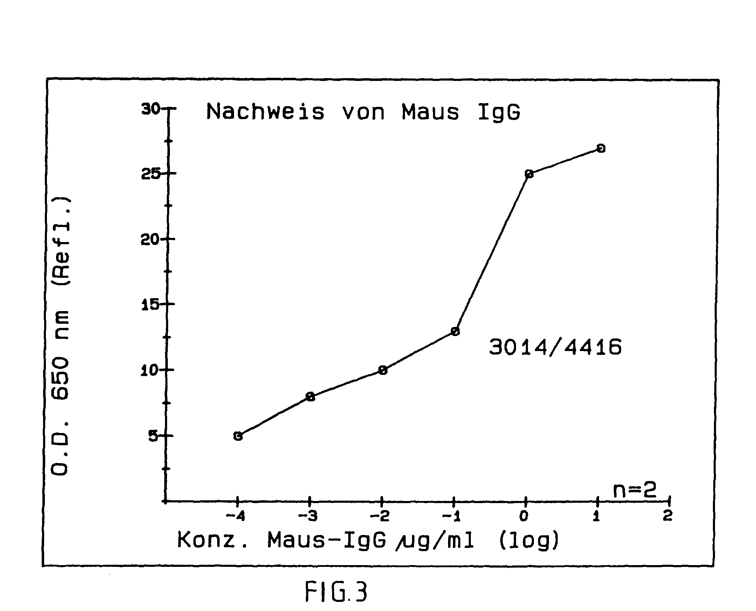

Die Ergebnisse der Bindungsexperimente sind wie folgt dargestellt (anhängende Fig. 1-3):
Fig. 1: System M 3014/I 5381/A 2028
Fig. 2: System M 3014/I 5381/A 4416
Fig. 3: System M 9637/I 5381/A 4416

Das Beispiel zeigt die hervorragende Eignung der erfindungsgemäßen Membranmatrizes für Immuntests. Der Analyt Maus IgG kann bis in den Nanogrammbereich sicher nachgewiesen werden.

Im Beispiel 2 wurden neben Wasser noch folgende Puffersysteme verwendet:

PBS (phosphatgepufferte physiologische Kochsalzlösung) 0,01 M Phosphat (= 0,008 M K₂HPO₄ und 0,002 M NaH₂PO₄) mit 0,15 M NaCl bei pH 7,4.

Acetat-Puffer Substratgemisch (für POD-Reaktion) 0,1 M Na-acetat in H₂O, pH 6,0
(eingestellt mit 1 M Citronensäure); als Redoxpartner wurde H₂O₂ (3,0 %) mit 1 ml je 1 zugegeben.

Tween 20 (Polyoxyethylensorbitan-monolaurat, Sigma Nr.: P 1379) 0,01 % in H₂O dest.

### Beispiel 3 (zum Vergleich)

### Durchführung eines Sandwich-ELISA zum Nachweis von Maus IgG

Beispiel 2 wurde wiederholt nur mit der Änderung, daß als Festphase keine erfindungsgemäße Polymerblend-Membran verwendet wurde, sondern eine Polyvinylidendifluoridmembran der Fa. Millipore (Immobilon P).

Die Membran wurde vor der in Beispiel 1 beschriebenen Testprozedur durch Durchsaugen von 100 µl Ethanol lyophilisiert. Da die Membran im Gegensatz zu den erfindungsgemäßen Polymerblend-Membranen kein Chromogen enthält, wurde sie nach der Immunreaktion in eine Substratlösung getaucht, die folgendermaßen hergestellt wurde:

Tetramethylbenzidin wurde in einer Konzentration von 2 mg/ml in Methanol gelöst (Stock-Lösung). Die Substratlösung wurde frisch aus 5 Teilen Stocklösung, 3,75 Teilen 0,4 M Citrat-Puffer (pH 5,5), 11,3 Teilen H₂O und 0,025 Teilen 30 % H₂O₂ hergestellt.

Die sich schnell zeigenden blauen Farbspots wurden immer größer, indifferenter und liefen teilweise ineinander über. Durch Ausbluten des Farbstoffs in die Substratlösung färbte sich diese stark blau und färbte so wiederum die gesamte Membran blau ein.

Nach der Trocknung der Membran war die Färbung außerdem nur wenige Minuten stabil, so daß keine reflektometrische Auswertung möglich war. Im Gegensatz dazu bleiben Farbspots auf den erfindungsgemäßen Polymer-Blendmembranen mehrere Tage, häufig bis zu einigen Wochen stabil und auswertbar.

### Beispiel 4

Immunologisch aktive Substanzen werden auf der Oberfläche der erfindungsgemäßen Polymer-Blendmembranen ausreichend fest immobilisiert. Die Immobilisierung kann durch Vorbehandlung mit Isocyanat-Bisulfitaddukten noch verbessert werden.

Diesen Effekt demonstriert das Beispiel 4. Ein Markerantikörper (Sigma A 9169, Typ: a-rabbit IgG, Ursprung: Ziege, Markierung: Peroxidase) wurde, wie in Beispiel 2 beschrieben, auf der Oberfläche einer Polymer-Blendmembran gemäß Beispiel 1 immobilisiert. In einem Fall war die Membran unbehandelt und im anderen mit einer 0,2%igen wäßrigen Lösung des Bisulfitaddukts vom Hexamethylendiisocyanat vorbehandelt. Die Vorbehandlung wurde so durchgeführt, daß die Membran mit der Bisulfitaddukt-Lösung durch Tauchen getränkt und die Membran anschließend bei 60°C 1 in einem Umlufttrockenschrank getrocknet wurde.

Nach Adsorption auf der Membranoberfläche wurde zur Überprüfung der Dauerhaftigkeit der Immobilisierung bis zu achtmal mit verschiedenen Puffern und Reagenzien gewaschen und nach den jeweiligen Waschvorgängen mittels Enzymreaktion der eingesetzten Peroxidase (POD) und TMB/H₂O₂ als Redox-Partner detektiert. In folgender Tabelle ist die Zahl der Waschvorgänge angegeben, nach denen man eine deutliche Abschwächung der Blaufärbung erkennen kann.

| | | | | | | |
|---|---|---|---|---|---|---|
| Medium | 1 | 2 | 3 | 4 | 5 | 6 |
| Membran unbehandelt | 4 | 6 | n.a. | n.a. | 4 | 3 |
| Membran vorbehandelt | n.a. | n.a. | n.a. | n.a. | 6 | 4 |
| n.a. = nicht ausgewaschen | | | | | | |

Verwendete Puffer und Reagenzien als Waschmedien:
- 1 =: H₂O
- 2 =: H₂O mit 0,1 % Tween 20
- 3 =: PBS
- 4 =: PBS mit 0,1 % Tween 20
- 5 =: Acetatpuffer
- 6 =: Carbonatpuffer
(0,1 M NaHCO₃ in H₂O, pH 9,6, eingestellt mit Natronlauge)

### Anhang:

Chemische Strukturen der bevorzugt eingesetzten Polymeren
Polyurethan (Desmoderm KBH®, Bayer AG)

Thermoplastisches Polyaddukt, welches durch Umsetzung von 75 Teilen eines Polyesters aus Adipinsäure, 70 Mol-% Ethylenglykol und 30 Mol-% 1,4-Butandiol (MG = 2000),
25 Teilen eines Polyesters aus Adipinsäure und 1,4 Butandiol (MG = 2250),
25 Teilen 1,4-Butandiol und
85 Teilen Diphenylmethandiisocyanat
erhalten wurde.

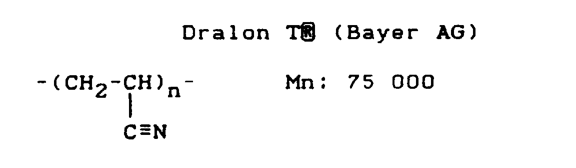

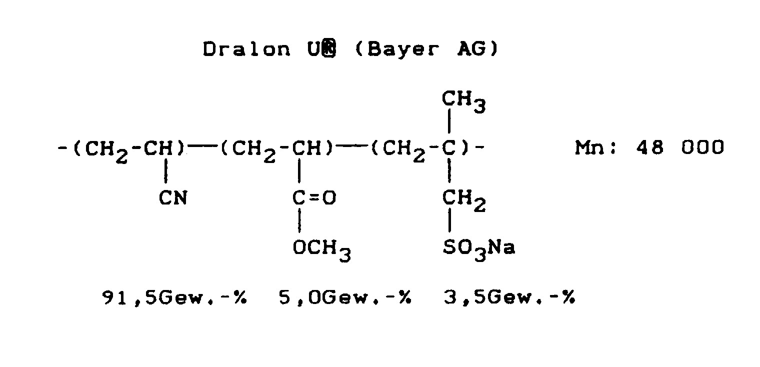

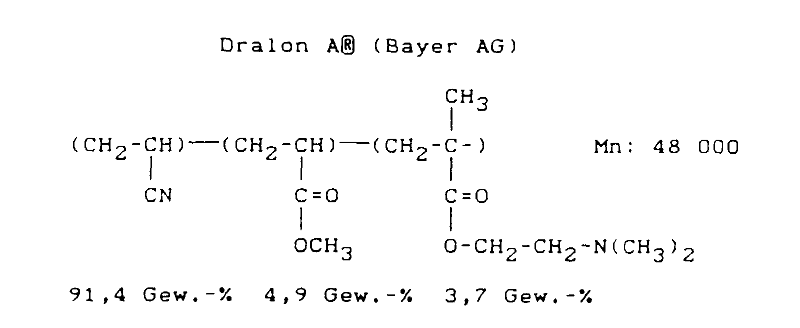

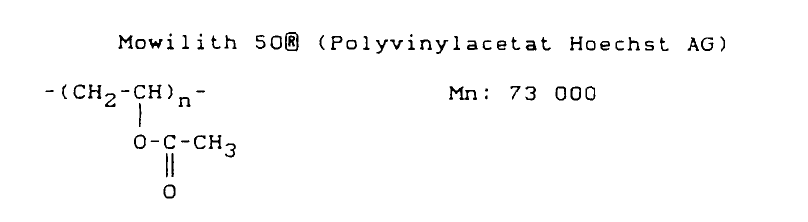

## Patentansprüche

1. Verfahren zur Durchführung immundiagnostischer Nachweise, bei welchen Enzyme oder Antikörper auf porösen Trägermaterialien gebunden werden, dadurch gekennzeichnet, daß als poröse Trägermaterialien makroporöse Polymermembranen eingesetzt werden, die hergestellt werden, indem man
a) in eine Lösung, die wenigstens zwei unverträgliche Polymere in solchen Mengen, die zu einer Phasentrennung der Lösung führen, enthält, unlösliche Füllstoffe eindispergiert, wobei eine homogene Gießlösung entsteht, und
b) diese Lösung auf einen Träger aufbringt und eine Fällungskoagulation durchführt,
wobei die Polymermembranen eine durch freie Aminogruppen modifizierte Oberfläche aufweisen, die durch den Zusatz von Polyethylenimin oder von aminmodifizierten Füllstoffen zur Gießlösung erhalten wird.

2. Verfahren nach Anspruch 1, dadurch gekennzeichnet, daß ein oder mehrere Füllstoffe aus der Gruppe von Talkum, Titandioxid, Bariumsulfat, Siliciumdioxid, mikrokristalline Zellulose, Zeolithe und Bentonite, hochdisperse Kieselsäuren und Quarz, die organisch modifiziert sein können, eingesetzt werden.

3. Verfahren nach Anspruch 1, dadurch gekennzeichnet, daß die Füllstoffe je nach spezifischer Oberfläche in Mengen von 5 bis 500 Gew.-%, bezogen auf das Gesamtgewicht der organischen Substanz der Membran, eingesetzt werden, wobei bei hochdispersen Füllstoffen vorzugsweise 5 bis 50 Gew.-% und bei Füllstoffen mit geringeren spezifischen Oberflächen vorzugsweise 35 bis 500 Gew.-% eingesetzt werden.

4. Verfahren nach Anspruch 1, dadurch gekennzeichnet, daß die wenigstens zwei unverträglichen Polymere ausgewählt sind aus den Kombinationen
a) Celluloseester/Polyvinylester,
b) Polyurethan/Polyacrylate bzw. Acrylcopolymere,
c) Polycarbonat-Copolymere/Polyurethan,
d) Polyvinylderivate/Polysulfone,
e) Polyamide bzw. Polyimide/Polystyrol bzw. Styrolcopolymere und
f) Poly-para-dimethyl-phenylenoxid/Polyvinylidenfluorid,
g) Polysulfon/Polyacrylnitril
h) Polyethersulfon / anionisch modifiziertes Polyacrylnitril
wobei weitere Kombinationen innerhalb der Polymerkombinationen möglich sind und wobei auch ternäre Polymergemische aus den genannten Polymeren eingesetzt werden können.

5. Verfahren nach Anspruch 1, dadurch gekennzeichnet, daß die makroporöse Polymermembran ein integriertes Chromogen, bevorzugt das 3,3',5,5'-Tetramethylbenzidin, enthält.

6. Verfahren nach Anspruch 1, dadurch gekennzeichnet, daß zur kovalenten Bindung der Enzyme oder Antikörper an eine makroporöse Membran mit einer durch freie Aminogruppen modifizierten Oberfläche Bisulfit-Addukte von Isocyanaten eingesetzt werden.

## Claims

1. Method for carrying out immunodiagnostic tests in which enzymes or antibodies are bound to porous carrier materials, characterized in that the porous carrier materials employed are macroporous polymer membranes which are prepared by
a) dispersing insoluble fillers in a solution which contains at least two incompatible polymers in amounts which lead to phase separation in the solution, resulting in a homogeneous casting solution, and
b) applying this solution to a support and carrying out a precipitation coagulation.
the polymer membranes having a surface which is modified by free amino groups and is obtained by the addition of polyethyleneimine or amine-modified fillers to the casting solution.

2. Method according to Claim 1, characterized in that one or more fillers from the group of talc, titanium dioxide, barium sulphate, silicon dioxide, microcrystalline cellulose, zeolites and bentonites, highly disperse silicas and quartz, each of which can be organically modified, are employed.

3. Method according to Claim 1, characterized in that the fillers are employed, depending on the specific surface area, in amounts of 5 to 500% by weight based on the total weight of the organic substance in the membrane, employing in the case of highly disperse fillers preferably 5 to 50% by weight and in the case of fillers with lower specific surface areas preferably 35 to 500% by weight.

4. Method according to Claim 1, characterized in that the at least two incompatible polymers are selected from the combinations of
a) cellulose esters/polyvinyl esters,
b) polyurethane/polyacrylates or acrylic copolymers,
c) polycarbonate copolymers/polyurethane,
d) polyvinyl derivatives/polysulphones,
e) polyamides or polyimides/polystyrene or styrene copolymers and
f) poly-para-dimethylphenylene oxide/polyvinylidene fluoride,
g) polysulphone/polyacrylonitrile
h) polyether sulphone/anionically modified polyacrylonitrile
with other combinations within the polymer combinations being possible and it also being possible to employ ternary polymer mixtures of the said polymers.

5. Method according to Claim 1, characterized in that the macroporous polymer membrane contains an integrated chromogen, preferably 3,3',5,5'-tetramethylbenzidine.

6. Method according to Claim 1, characterized in that bisulphite adducts of isocyanates are employed for the covalent bonding of the enzymes or antibodies to a macroporous membrane with a surface modified by free amino groups.

## Revendications

1. Procédé de mise en oeuvre d'essais immunodiagnostiques, dans lequel des enzymes ou anticorps sont liés à des matériaux support poreux, caractérisé en ce que l'on met en oeuvre comme matériau support poreux, des membranes polymères macroporeuses, qui sont préparées en ce que/
a) l'on disperse une charge insoluble dans une solution qui contient au moins deux polymères incompatibles en des quantités telles que cela conduit à une séparation de phases dans la solution, ce par quoi il se forme une solution de coulée homogène, et
b) on applique cette solution sur un support et une coagulation par précipitation est réalisée,
où les membranes de polymères présentent une surface modifiée par des radicaux amino libres, qui est obtenue par addition de polyéthylène-imines ou de charges amino-modifiées à la solution de coulée.

2. Procédé suivant la revendication 1, caractérisé en ce que l'on met en oeuvre une ou plusieurs charges du groupe des talc, dioxyde de titane, sulfate de baryum, dioxyde de silicium, cellulose microcristalline, zéolite et bentonite, acide silicique bien dispersé et quartz, qui peuvent être organiquement modifiés.

3. Procédé suivant la revendication 1, caractérisé en ce que la charge est mise en oeuvre, suivant la surface spécifique, en des quantités allant de 5 à 500% en poids, sur base du poids total de la substance organique de la membrane, où pour des charges bien dispersées, on met en oeuvre, de préférence, 5 à 50% en poids et pour des charges avec des surfaces spécifiques plus faibles, de préférence 35 à 500% en poids.

4. Procédé suivant la revendication 1, caractérisé en ce que les au moins deux polymères incompatibles sont choisis parmi les combinaisons :
a) ester de cellulose/poly(ester vinylique);
b) polyuréthanne/polyacrylate ou copoplymère acrylique;
c) copolymère de polycarbonate/polyuréthanne;
d) dérivé polyvinylique/polysulfone;
e) polyamide ou polyimide/polystyrène ou copolymère de styrène, et
f) poly(oxyde de p-diméthylphénylène)/ poly(fluorure de vinylidène);
g) polysulfone/polyacrylonitrile;
h) polyéthersulfone/polyacrylonitrile anioniquement modifié,
où d'autres combinaisons sont possibles au sein des combinaisons de polymères et où des mélanges tertiaires de polymères peuvent également être mis en oeuvre à partir des polymères cités.

5. Procédé suivant la revendication 1, caractérisé en ce que la membrane polymère macroporeuse contient un chromogène intégré, de préférence, la 3,3',5,5'-tétraméthylbenzidine.

6. Procédé suivant la revendication 1, caractérisé en ce que, pour la liaison covalente de l'enzyme ou de l'anticorps sur une membrane macroporeuse avec une surface modifiée par des radicaux amino libres, on met en oeuvre un adduit bisulfite d'isocyanate.
